# EUROPEAN PATENT APPLICATION

(11) **EP 3 175 767 A1**
(43) Date of publication of application: **07.06.2017**
(21) Application number: 15827800.2
(22) Date of filing: 29.07.2015
(51) Int. Cl.: A61B 1/00, A61B 1/04, G02B 23/24

(54) **CONTROL DEVICE AND ENDOSCOPE SYSTEM**

(30) Priority: 01.08.2014 JP 2014157649
(71) Applicant: Olympus Corporation, Tokyo 192-8507 (JP)
(72) Inventor: KUTSUMA, Yuji, Hachioji-shi Tokyo 192-8507 (JP); SUZUKI, Tatsuhiko, Hachioji-shi Tokyo 192-8507 (JP); IWASAKI, Tomoki, Hachioji-shi Tokyo 192-8507 (JP); HASHIMOTO, Susumu, Hachioji-shi Tokyo 192-8507 (JP); HAMADA, Toshihiro, Hachioji-shi Tokyo 192-8507 (JP)
(74) Representative: Gunzelmann, Rainer
(86) International application number: PCT/JP2015/071526
(87) International publication number: WO 2016/017704

(57) **Abstract**

User-friendly control device and endoscope system are provided. A control device 6 includes an acquisition unit 661 which acquires identification information including switch information indicating the number of switches provided on an endoscope 2 and function information indicating at least any one of a function which the endoscope 2 may execute and a function which the endoscope 2 cannot execute from the endoscope 2, a setting information recording unit 641 which records setting information in which a predetermined function is assigned to each switch of the endoscope 2 connected to the control device 6, and a setting unit 662 which generates function setting information in which the function to be executed is assigned to be set according to a signal output from each of a plurality of switches of the endoscope 2 connected to the control device 6 based on the identification information acquired by the acquisition unit 661 and the setting information recorded by the setting information recording unit 641.

## Description

### Field

The present invention relates to a control device configured to be connected to an endoscope that is configured to be inserted into a subject and image a body cavity of the subject to generate image data. The present invention also relates to an endoscope system.

### Background

Conventionally, technology of assigning functions of capturing and printing to each of a plurality of switches provided on an endoscope according to the endoscope to be connected by a control device (processor) to which the endoscope is connected is known (refer to Patent Literature 1). In this technology, a memory which records identification of each of a plurality of endoscopes, identification information of a user of each endoscope, and setting information in which a function of each switch set for each identification information of the user is assigned is provided on the control device, and when the endoscope is connected to the control device, a signal output from each switch of the endoscope is automatically assigned to the function based on the identification information of the connected endoscope and the identification information of the user of the endoscope.

### Citation List

### Patent Literature

Patent Literature 1: JP 2007-313132 A

### Summary

### Technical Problem

However, since the setting information should be provided for each endoscope and each user in Patent Literature 1 described above, the user should create the setting information each time the number of users increases and the number of endoscopes increases, which is not user friendly.

The present invention has been made in view of the foregoing, and an object of the invention is to provide user-friendly control device and endoscope system. Solution to Problem

In order to solve the above described problem and achieve the object, a control device according to the invention is configured to be connected to an endoscope having a plurality of switches to be operated to output signals. The control device includes: an acquisition unit configured to acquire, from the endoscope connected to the control device, identification information that contains switch information indicating the number of the switches of the endoscope and contains function information indicating at least one of a function that is executable by the endoscope and a function that is not executable by the endoscope; a recording unit configured to record setting information that assigns predetermined functions to be executed corresponding to the signals respectively output from the plurality of switches of the endoscope connected to the control device; a setting unit configured to generate function setting information that assigns and sets functions to be executed corresponding to the signals respectively output from the plurality of switches of the endoscope connected to control device, based on the identification information acquired by the acquisition unit and the setting information recorded by the recording unit; and an executing unit configured to, when one of the signals is output from a switch of the plurality of switches, execute one of the functions assigned to the switch which outputs the one of the signals according to the function setting information generated by the setting unit.

In the control device according to the above-described invention, if the identification information does not contain one of the predetermined functions assigned in the setting information, the setting unit is configured to assign and set a function frequently used in the control device to a switch of the plurality of switches to which a function not including the one of the predetermined functions is to be assigned.

In the control device according to the above-described invention, the identification information further contains type information of the endoscope; the acquisition unit is configured to acquire, from the endoscope, the type information of the endoscope contained in the identification information; and if the identification information does not contain one of the predetermined functions assigned in the setting information, the setting unit is configured to assign and set a function of high importance according to the type information of the endoscope, to a switch of the plurality of switches to which a function not including the one of the predetermined functions is to be assigned.

The control device according to the above-described invention further includes a generating unit configured to generate information indicating correspondence relationship between the plurality of switches and the functions of the switches assigned by the setting unit.

An endoscope system according to the invention includes: the above-described control device; and a display device configured to display the information generated by the generating unit.

### Advantageous Effects of Invention

According to the present invention, it is possible to improve user friendliness.

### Brief Description of Drawings

FIG. 1 is a functional block diagram of main parts of an endoscope system according to a first embodiment of the present invention.
FIG. 2 is an exemplary diagram of identification information recorded by an identification information recording unit of an endoscope illustrated in FIG. 1.
FIG. 3 is an exemplary diagram of an image displayed on a display device illustrated in FIG. 1.
FIG. 4 is an exemplary diagram of setting information recorded by a setting information recording unit of a control device illustrated in FIG. 1.
FIG. 5 is a flowchart illustrating an outline of a process executed by the endoscope device according to the first embodiment of the present invention.
FIG. 6 is an exemplary diagram of function setting information generated by a setting unit of the control device illustrated in FIG. 1.
FIG. 7 is an exemplary diagram of the image displayed on the display device illustrated in FIG. 1.
FIG. 8 is a functional block diagram of main parts of an endoscope system according to a second embodiment of the present invention.
FIG. 9 is a flowchart illustrating an outline of a process executed by the endoscope device according to the second embodiment of the present invention.
FIG. 10 is an exemplary diagram of function setting information generated by a setting unit of a control device illustrated in FIG. 8.
FIG. 11 is an exemplary diagram of an image displayed on a display device illustrated in FIG. 8.

### Description of Embodiments

Modes for carrying out the present invention (hereinafter, referred to as "embodiment(s)") will be hereinafter described. Exemplary embodiments include a medical endoscope system for capturing an in-vivo image of a subject, such as a patient, to display the in-vivo image. The present invention is not limited by the following embodiments. The same reference signs are used to designate the same elements throughout the drawings.

### (First Embodiment)

### [Configuration of Endoscope System]

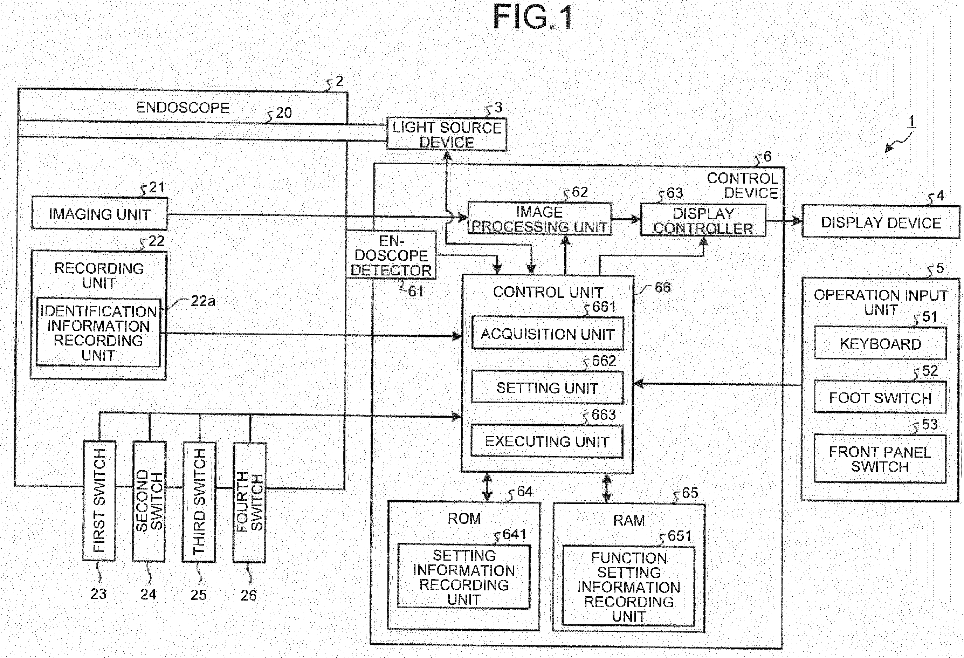

FIG. 1 is a functional block diagram of main parts of an endoscope system according to a first embodiment of the present invention.

An endoscope system 1 illustrated in FIG. 1 is provided with an endoscope 2 (endoscope scope) which captures an in-vivo image of a subject with a distal end portion inserted into a body cavity of the subject, a light source device 3 which generates illumination light emitted from a distal end of the endoscope 2, a display device 4 which displays an image corresponding to data of the image captured by the endoscope 2, an operation input unit 5 which accepts an input of various operations of the endoscope system 1, and a control device 6 (processor) which performs predetermined image processing on the in-vivo image captured by the endoscope 2 and comprehensively controls operation of an entire endoscope system 1.

First, a configuration of the endoscope 2 will be described.

The endoscope 2 is provided with a light guide path 20 which propagates the illumination light emitted by the light source device 3, an imaging unit 21 which captures an image of an object, for example, an image in the body cavity of the subject to generate the image data, a recording unit 22 which records identification information for identifying the endoscope 2, and first to fourth switches 23 to 26 pressed to output signals.

The light guide path 20 formed of an illumination lens and a light guide propagates the illumination light emitted by the light source device 3 to a predetermined area.

The imaging unit 21 includes an optical system having one or more lenses, a prism and the like, and an image sensor such as a CCD (charge coupled device) and a CMOS (complementary metal oxide semiconductor) which performs photoelectric conversion on light collected by the optical system and generates an electric signal as an image signal. The imaging unit 21 transmits the image signal to the control device 6.

The recording unit 22 records various operations and information of the endoscope 2. The recording unit 22 includes an identification information recording unit 22a. The identification information recording unit 22a records the identification information for identifying the endoscope 2.

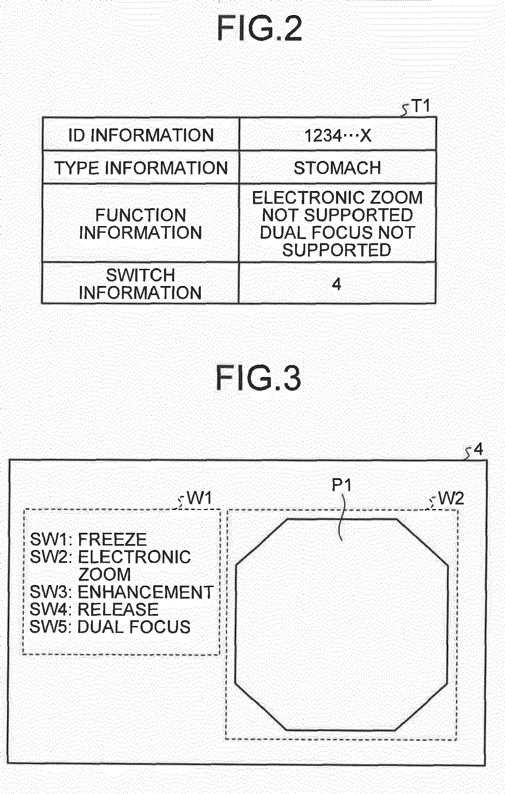

FIG. 2 is an exemplary diagram of the identification information recorded by the identification information recording unit 22a. As illustrated in FIG. 2, identification information T1 contains ID information of the endoscope 2, switch information indicating the number of switches provided on the endoscope 2, function information indicating at least one of a function that is executable by the endoscope 2 and a function that is not executable by the endoscope 2, and type information of the endoscope 2. Specifically, in the identification information T1, the ID information (serial number) of the endoscope 2 reads "1234...X", the type information of the endoscope 2 reads "stomach", the function information reads "electronic zoom not supported" and "DUAL FOCUS not supported", and the switch information reads "four" (no fifth switch exists). The identification information T1 may also contain a model year and a model name of the endoscope 2, specification information such as CCD or CMOS, locations of the switches, a transmission method, and a transmission rate.

Each of the first to fourth switches 23 to 26 formed of a pull switch or a slide switch transmits the signal to the control device 6 according to pressure from outside.

Next, the light source device 3 will be described.

The light source device 3 is formed of a light source such as a xenon lamp and an LED, a lens which condenses light emitted by the light source, and a filter which transmits only light of a predetermined wavelength band. The light source device 3 emits the illumination light out of the endoscope 2 through the light guide path 20.

Next, the display device 4 will be described.

The display device 4 displays the image corresponding to the image data input from the control device 6. The display device 4 is formed of a display panel of liquid crystal, organic EL (electro luminescence) and the like.

FIG. 3 is an exemplary diagram of the image displayed on the display device 4. As illustrated in FIG. 3, the display device 4 includes a display area W1 for displaying an image P1 corresponding to the image data captured by the endoscope 2 and a switch display area W2 for displaying an image corresponding to information indicating correspondence relationship between each switch of the endoscope 2 and a function of each switch assigned by a setting unit 662 of a control unit 66 to be described later.

Next, the operation input unit 5 will be described.

The operation input unit 5 is provided with a keyboard 51 which accepts an input of various signals, a foot switch 52 which outputs a signal according to operation of an operator, and a front panel switch 53 which accepts an input of various signals.

Next, reference will be made to the control device 6.

The control device 6 includes an endoscope detector 61, an image processing unit 62, a display controller 63, a ROM (read only memory) 64, a RAM (random access memory) 65, and a control unit 66.

The endoscope detector 61 detects the endoscope 2 connected to the control device 6 and outputs a detection result to the control unit 66. The endoscope detector 61 is formed of a pull switch and the like.

The image processing unit 62 performs the predetermined image processing on the image data input from the imaging unit 21 of the endoscope 2 to output to the display controller 63. Herein, the predetermined image processing includes synchronization processing, optical black reduction processing, white balance adjustment processing, color matrix operation processing, gamma correction processing, color reproduction processing, edge enhancement processing, format conversion processing and the like, for example. The image processing unit 62 generates the information indicating the correspondence relationship between each of the first to fourth switches 23 to 26 of the endoscope 2 and the function assigned to each switch by the setting unit 662 of the control unit 66 to be described later. In this sense, the image processing unit 62 serves as a generating unit in the first embodiment.

The display controller 63 allows the display device 4 to display the image data on which the image processing is performed by the image processing unit 62. This allows the display device 4 to display the information indicating the correspondence relationship between each of the first to fourth switches 23 to 26 of the endoscope 2 and the function assigned to each switch by the setting unit 662 of the control unit 66 to be described later generated by the image processing unit 62.

The ROM 64 records various pieces of information of the control device 6, a program executed by the control device 6 and the like. The ROM 64 includes a setting information recording unit 641 which records setting information in which a predetermined function to be executed corresponding to the signal output from each switch unit of the endoscope 2 is assigned.

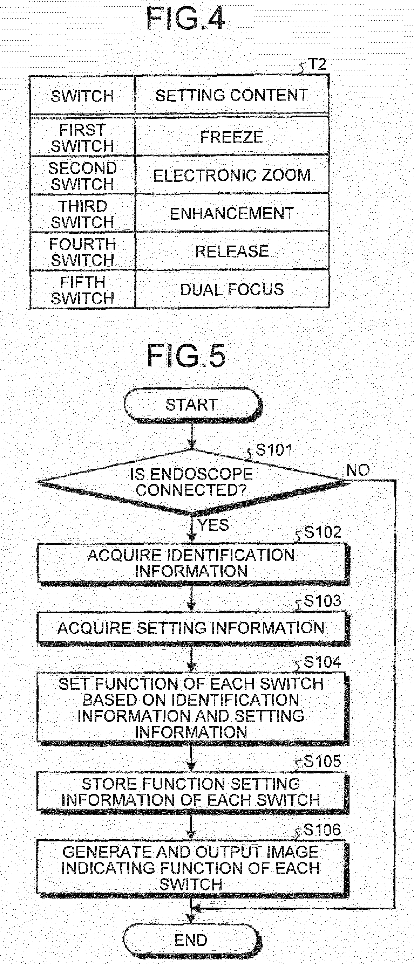

FIG. 4 is an exemplary diagram of the setting information (user preset) recorded by the setting information recording unit 641. In setting information T2 illustrated in FIG. 4, the information in which the predetermined function to be executed corresponding to the signal output from each switch of the endoscope 2 connected to the control device 6 is assigned by a user through the keyboard 51 is recorded. Specifically, "freeze" to freeze (stop) the image displayed on the display device 4 is assigned to the function to be executed corresponding to the signal output from the first switch 23, "electronic zoom" is assigned to the function to be executed corresponding to the signal output from the second switch 24, "enhancement" to enhance a contour of the image is assigned to the function to be executed corresponding to the signal output from the third switch 25, "release" to instruct to capture is assigned to the function to be executed corresponding to the signal output from the fourth switch 26, and "DUAL FOCUS" to change a focal distance is assigned to the function to be executed corresponding to the signal output from the fifth switch.

The RAM 65 temporarily records information used by the control device 6. The RAM 65 includes a function setting information recording unit 651 which temporarily records function setting information of each switch set by the setting unit 662 to be described later.

The control unit 66 performs driving control of each component including the imaging unit 21 of the endoscope 2 and the light source device 3, input/output control on each component and the like. The control unit 66 includes an acquisition unit 661, the setting unit 662, and an executing unit 663.

The acquisition unit 661 acquires the identification information of the endoscope 2 connected to the control device 6 from the identification information recording unit 22a of the endoscope 2.

The setting unit 662 generates the function setting information in which the predetermined function to be executed corresponding to the signal output from each of a plurality of switches provided on the endoscope 2 connected to the control device 6 is assigned, the function setting information corresponding to the endoscope 2 connected to the control device 6 based on the identification information acquired by the acquisition unit 661 and the setting information recorded by the setting information recording unit 641 of the ROM 64. The function setting information generated by the setting unit 662 is temporarily recorded by the function setting information recording unit 651 of the RAM 65.

When the signal is output from any one of the first to fourth switches 23 to 26 of the endoscope 2 connected to the control device 6, the executing unit 663 executes the function assigned to the switch which outputs the signal according to the function setting information recorded by the function setting information recording unit 651.

### [Process of Endoscope System]

Next, reference will be made to a process executed by the endoscope system 1. FIG. 5 is a flowchart illustrating an outline of the process executed by the endoscope system 1. In FIG. 5, the process executed when the endoscope 2 is connected to the control device 6 will be described. In the following example, the endoscope 2 to be connected to the control device 6 has the first to fourth switches 23 to 26. It goes without saying that the endoscope 2 having the first switch 23 to a fifth switch may be employed.

As illustrated in FIG. 5, when the endoscope detector 61 detects connection of the endoscope 2 (step S101: Yes), the acquisition unit 661 acquires the identification information from the identification information recording unit 22a of the connected endoscope 2 (step S102).

Subsequently, the setting unit 662 acquires the setting information from the setting information recording unit 641 (step S103) and generates the function setting information to set the function of each switch of the endoscope 2 based on the identification information acquired by the acquisition unit 661 and the setting information (step S104).

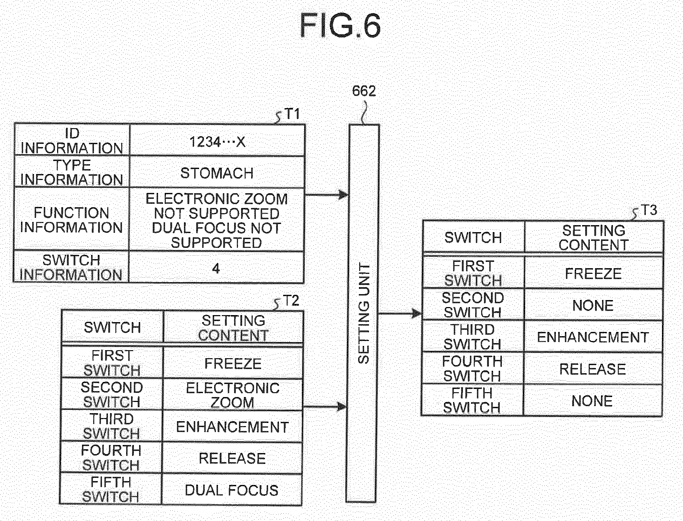

FIG. 6 is an exemplary diagram of the function setting information generated by the setting unit 662. As illustrated in FIG. 6, the setting unit 662 generates function setting information T3 based on the identification information T1 and setting information T2. Specifically, as illustrated in FIG. 6, since the connected endoscope 2 does not support the electronic zoom and does not include the fifth switch, the setting unit 662 generates the function setting information T3 in which the function is not assigned to SW2 (second switch 24) and the function is not assigned to SW5.

Thereafter, the setting unit 662 stores the function setting information of each switch in the function setting information recording unit 651 of the RAM 65 (step S105).

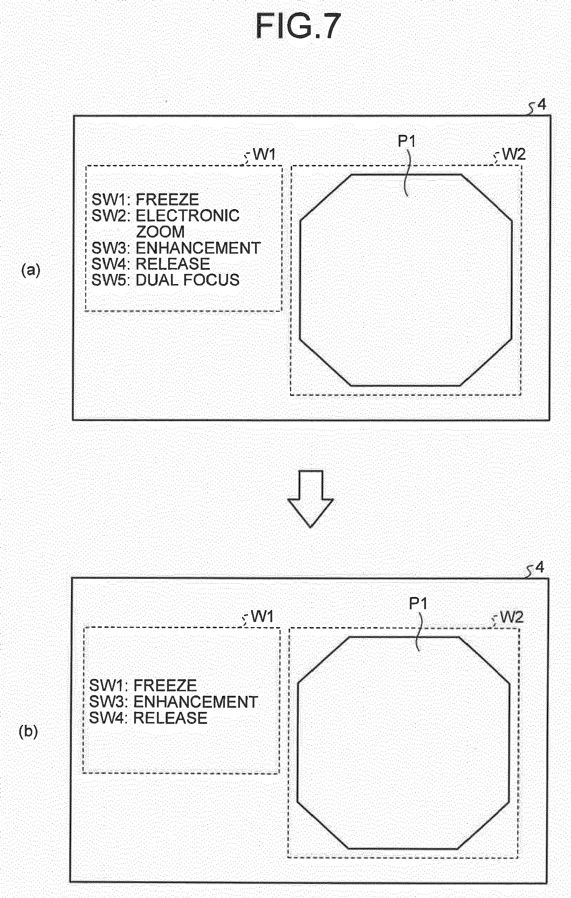

Subsequently, the image processing unit 62 generates the image indicating the function of each switch based on the function setting information recorded by the function setting information recording unit 651 and outputs the image to the display device 4 (step S106). According to this, as illustrated in FIG. 7, the display device 4 displays the function assigned to each switch (FIG. 7(a) to FIG. 7(b)). As a result, the user may intuitively grasp the function assigned to each switch in the endoscope 2 connected to the control device 6. Furthermore, since the switch to which the function is not assigned is not displayed on the display device 4, erroneous switch operation may be prevented. After step S106, the endoscope system 1 finishes this process. After this process, the user starts examining the body cavity of the subject.

At step S101, when the endoscope detector 61 does not detect the connection of the endoscope 2 (step S101: No), the endoscope system 1 finishes this process.

According to the first embodiment described above, since the function setting information set by the setting unit 662 by assigning the function to be executed corresponding to the signal output from each of a plurality of switches provided on the endoscope 2 connected to the control device 6 based on the identification information acquired by the acquisition unit 661 and the setting information recorded by the setting information recording unit 641 is temporarily generated each time the endoscope 2 is connected, the setting information set in advance by the user is not changed and the user is not required to create the setting information corresponding to the endoscope 2 connected to the control device 6 each time, so that user-friendliness can be improved.

Furthermore, according to the first embodiment, the display device 4 displays the function assigned to each switch. As a result, the operator may intuitively grasp the function assigned to each switch in the endoscope 2 connected to the control device 6.

### (Second Embodiment)

Next, reference will be made to a second embodiment of the present invention. An endoscope system according to the second embodiment has a configuration similar to that of the endoscope system 1 according to the first embodiment described above but includes a control device with a different configuration. Therefore, hereinafter, the same reference signs are used to designate the same elements as those of the endoscope system 1 according to the first embodiment described above, and the explanation thereof will be omitted.

### [Configuration of Endoscope System]

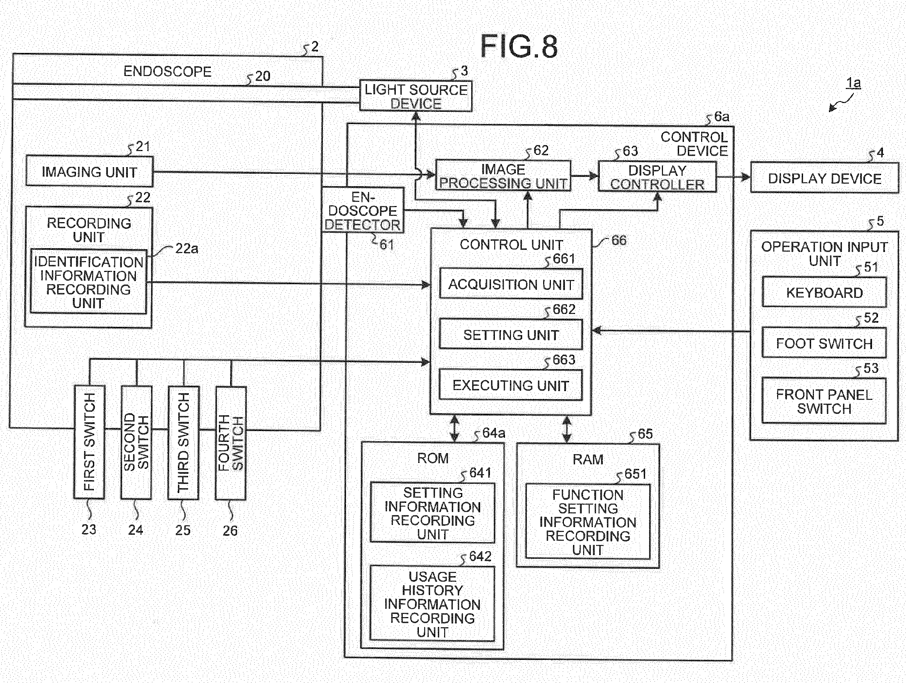

FIG. 8 is a functional block diagram of main parts of the endoscope system according to the second embodiment. An endoscope system 1a illustrated in FIG. 8 is provided with a control device 6a in place of the control device 6 according to the first embodiment described above.

The control device 6a is provided with a ROM 64a in place of the ROM 64 according to the first embodiment described above. The ROM 64a records various pieces of information of the control device 6a, a program executed by the control device 6a and the like. The ROM 64a includes a setting information recording unit 641 and a usage history information recording unit 642 which records usage history information indicating a history of a function used in the endoscope 2.

### [Process of Endoscope System]

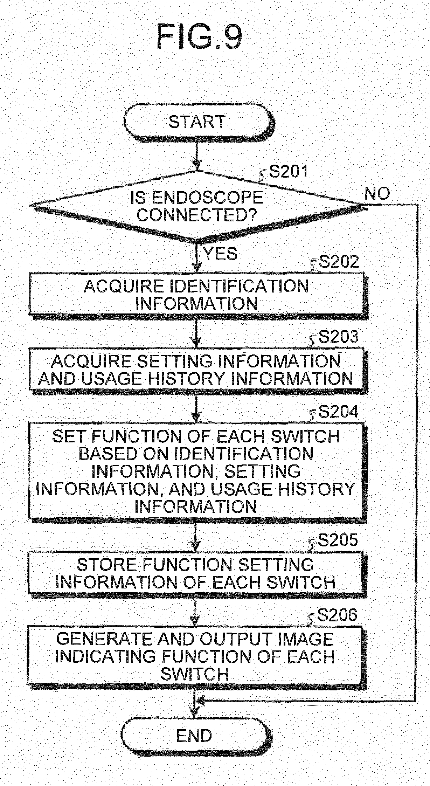

Next, reference will be made to a process executed by the endoscope system 1a. FIG. 9 is a flowchart illustrating an outline of the process executed by the endoscope system 1a. In FIG. 9, the process executed when the endoscope 2 is connected to the control device 6a will be described. In the following example, the endoscope 2 to be connected to the control device 6a has first to fourth switches 23 to 26.

Steps S201 and S202 correspond to steps S101 and S102 in FIG. 5 described above, respectively.

Subsequently, a setting unit 662 acquires setting information and the usage history information from the setting information recording unit 641 and usage history information recording unit 642, respectively (step S203).

Thereafter, the setting unit 662 generates function setting information in which a function to be executed according to a signal output from each switch of the endoscope 2 is assigned to be set, the function setting information of the endoscope 2 connected to the control device 6a based on identification information acquired by an acquisition unit 661, the setting information, and the usage history information (step S204).

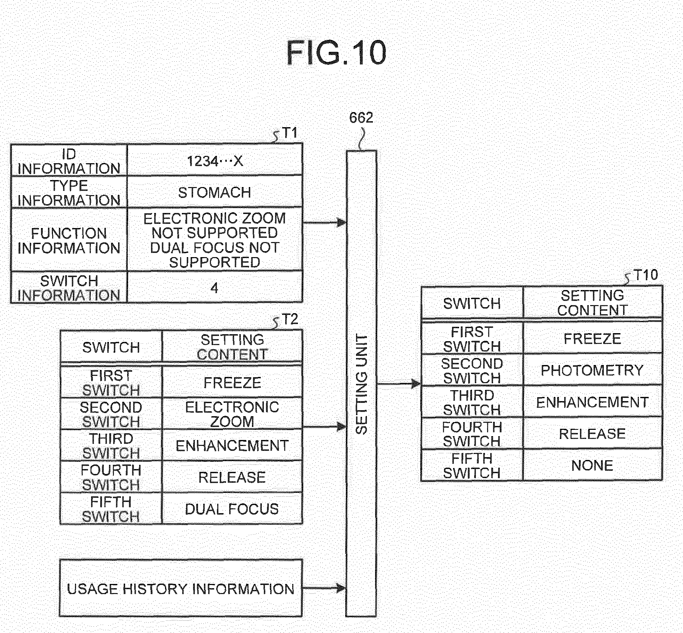

FIG. 10 is an exemplary diagram of the function setting information generated by the setting unit 662. As illustrated in FIG. 10, the setting unit 662 generates function setting information T10 based on identification information T1, setting information T2, and the usage history information. Specifically, if the identification information T1 does not contain a predetermined function assigned in the setting information T2, the setting unit 662 assigns and sets a function frequently used in the endoscope 2 to the switch to which the function not including the predetermined function is to be assigned. For example, as illustrated in FIG. 10, since the connected endoscope 2 does not support electronic zoom and does not include a fifth switch, the setting unit 662 generates the function setting information T10 in which the function of SW2 (second switch 24) is changed from the electronic zoom to photometry that is frequently used in the endoscope 2 based on the usage history information and no function is assigned to SW5.

Subsequently, the setting unit 662 stores function information of each switch in a function setting information recording unit 651 of a RAM 65 (step S205).

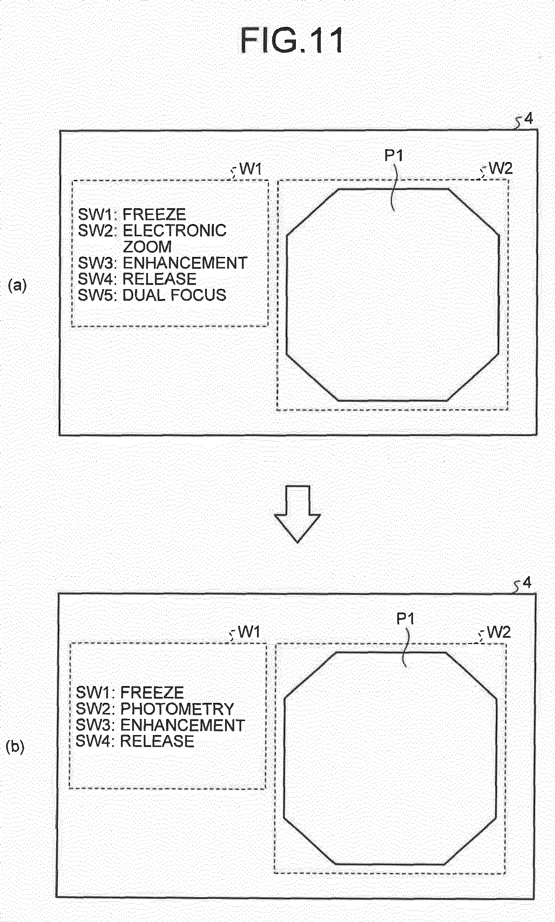

Thereafter, the image processing unit 62 generates an image indicating the function of each switch based on the function setting information recorded by the function setting information recording unit 651 and outputs the image to a display device 4 (step S206). According to this, as illustrated in FIG. 11, the display device 4 displays the function assigned to each switch (FIG. 11(a) to FIG. 11(b)). As a result, an operator may intuitively grasp the function assigned to each switch in the endoscope 2 connected to the control device 6a. After step S206, the endoscope system 1a finishes this process. After this process, the operator starts examining a body cavity of a subject.

According to the second embodiment described above, if the identification information does not contain the predetermined function assigned in the setting information, the setting unit 662 assigns a function of high importance in the control device 6a to the switch to which the function not including the predetermined function is to be assigned, thereby assigns the function to each of the plurality of switches of the endoscope 2 connected to the control device 6a, based on the identification information acquired by the acquisition unit 661, the setting information recorded by the setting information recording unit 641, and the usage history information, so that user-friendliness can be further improved.

In the second embodiment, if the identification information does not contain the predetermined function assigned in the setting information, the setting unit 662 assigns the function frequently used in the control device 6a to the switch to which the function not including the predetermined function is to be assigned, based on the identification information, setting information, and usage history information. However, it is also possible to set the frequently used function in advance in the setting information in addition to the function of each switch, for example.

In the second embodiment, the setting unit 662 sets functions to be assigned to the plurality of switches of the endoscope 2 connected to the control device 6a based on the identification information acquired by the acquisition unit 661, the setting information recorded by the setting information recording unit 641, and the frequently used function (frequently used). Instead of the frequently used function, it is also possible to preferentially assign a function of high importance according to a type of the endoscope 2 connected to the control device 6a. Specifically, if the identification information T1 does not contain the predetermined function assigned in the setting information T2, the setting unit 662 may assign and set the function of high importance according to type information of the endoscope 2 to the switch to which the function not including the predetermined function is to be assigned. Thus, the user-friendliness can be improved.

### (Other Embodiments)

Although the setting unit generates the function setting information in which the function is assigned to be set to each of a plurality of switches provided on the endoscope connected to the control device based on the identification information and setting information in the first and second embodiments, when the endoscope having a specific function such as DUAL FOCUS, AFI observation, and NBI observation is connected to the control device, for example, it is also possible to generate the function setting information in which the specific function is preferentially assigned to the first and second switches.

In this manner, the present invention may include various embodiments not herein described and various design changes and the like may be made within the scope of the technical idea specified by claims.

### Reference Signs List

- 1, 1a: ENDOSCOPE SYSTEM
- 2: ENDOSCOPE
- 3: LIGHT SOURCE DEVICE
- 4: DISPLAY DEVICE
- 5: OPERATION INPUT UNIT
- 6, 6a: CONTROL DEVICE
- 20: LIGHT GUIDE PATH
- 21: IMAGING UNIT
- 22: RECORDING UNIT
- 22a: IDENTIFICATION INFORMATION RECORDING UNIT
- 23: FIRST SWITCH
- 24: SECOND SWITCH
- 25: THIRD SWITCH
- 26: FOURTH SWITCH
- 51: KEYBOARD
- 52: FOOT SWITCH
- 53: FRONT PANEL SWITCH
- 61: ENDOSCOPE DETECTOR
- 62: IMAGE PROCESSING UNIT
- 63: DISPLAY CONTROLLER
- 64, 64a: ROM
- 65: RAM
- 66: CONTROL UNIT
- 641: SETTING INFORMATION RECORDING UNIT
- 642: USAGE HISTORY INFORMATION RECORDING UNIT
- 651: FUNCTION SETTING INFORMATION RECORDING UNIT
- 661: ACQUISITION UNIT
- 662: SETTING UNIT
- 663: EXECUTING UNIT
- P1: IMAGE
- T1: IDENTIFICATION INFORMATION
- T2: SETTING INFORMATION
- T3, T10: FUNCTION SETTING INFORMATION

## Claims

1. A control device configured to be connected to an endoscope having a plurality of switches to be operated to output signals, the control device comprising:
an acquisition unit configured to acquire, from the endoscope connected to the control device, identification information that contains switch information indicating the number of the switches of the endoscope and contains function information indicating at least one of a function that is executable by the endoscope and a function that is not executable by the endoscope;
a recording unit configured to record setting information that assigns predetermined functions to be executed corresponding to the signals respectively output from the plurality of switches of the endoscope connected to the control device;
a setting unit configured to generate function setting information that assigns and sets functions to be executed corresponding to the signals respectively output from the plurality of switches of the endoscope connected to control device, based on the identification information acquired by the acquisition unit and the setting information recorded by the recording unit; and
an executing unit configured to, when one of the signals is output from a switch of the plurality of switches, execute one of the functions assigned to the switch which outputs the one of the signals according to the function setting information generated by the setting unit.

2. The control device according to claim 1, wherein
if the identification information does not contain one of the predetermined functions assigned in the setting information, the setting unit is configured to assign and set a function frequently used in the control device to a switch of the plurality of switches to which a function not including the one of the predetermined functions is to be assigned.

3. The control device according to claim 1, wherein:
the identification information further contains type information of the endoscope;
the acquisition unit is configured to acquire, from the endoscope, the type information of the endoscope contained in the identification information; and
if the identification information does not contain one of the predetermined functions assigned in the setting information, the setting unit is configured to assign and set a function of high importance according to the type information of the endoscope, to a switch of the plurality of switches to which a function not including the one of the predetermined functions is to be assigned.

4. The control device according to claim 1, further comprising a generating unit configured to generate information indicating correspondence relationship between the plurality of switches and the functions of the switches assigned by the setting unit.

5. An endoscope system comprising:
the control device according to claim 4; and
a display device configured to display the information generated by the generating unit.
